# EUROPEAN PATENT APPLICATION

(11) **EP 1 407 740 A1**
(43) Date of publication of application: **14.04.2004**
(21) Application number: 02771748.7
(22) Date of filing: 21.05.2002
(51) Int. Cl.: A61F 13/15

(54) **INTERLABIAL PAD INDIVIDUAL PACKAGING BODY**

(30) Priority: 22.05.2001 JP 2001152403; 05.03.2002 JP 2002059534
(71) Applicant: Uni-Charm Corporation, Kawanoe-shi, Ehime-ken 799-0111 (JP)
(72) Inventor: MIZUTANI, S., c/o Technical Center, Uni-Charm Co., Mitoyo-gun, Kagawa 769-1602 (JP); YAMAKI, K., c/o Technical Center, Uni-Charm Co., Kagawa 769-1602 (JP); NODA, Y., c/o Technical Center, Uni-Charm Co., Kagawa 769-1602 (JP); TOKUMOTO, M., c/o Technical Center, Uni-Charm Co., Kagawa 769-1602 (JP); SAKAI, A., c/o Technical Center, Uni-Charm Co., Kagawa 769-1602 (JP)
(74) Representative: Purdy, Hugh Barry
(86) International application number: PCT/JP2002/004887
(87) International publication number: WO 2002/094151

(57) **Abstract**

The present invention relates to a wrapping body to be a member to discard an used interlabial pad, composed of a wrapping container to wrap the interlabial pad to provide the wrapping body where the interlabial pad is contained in the wrapping container realizing hygienic and smooth discards of used interlabial pads and compactness when carried.

Take out the interlabial pad (10) where the unused interlabial pad (10) is wrapped in the sheet (2) completely by opening the folding area of the wrapping body (1) and unfolding the same into a flat shape. Then wrap the used interlabial pad (20) with the unfolded sheet (2) to discard it.

## Description

### Background of the Invention

### Technical Field

The present invention relates to a wrapping body composed of an individual interlabial pad wrapping container to be a discarding member of a used interlabial pad.

### Background Art

Conventionally, a sanitary napkin and a tampon are used generally as a sanitary product for female. Here, there have been great efforts to prevent the leak of menstrual blood from a gap caused by poor adhesion near the ostium vaginae as for the sanitary napkin. Moreover, as for the tampon, there have been great efforts for relieving the foreign feeling and the discomfort when wearing the sanitary products and intervaginal wearing trouble due to the nature of those products.

Under such situation, a sanitary product of the interlabial pad have attracted people as the sanitary product positioned between the sanitary napkin and the tampon in recent years.

The interlabial pad is fixed by inserting it between the labia, having characteristics that it is difficult to cause the leak of menstrual blood since it has higher adhesion to the body than that of the sanitary napkin and since psychological resistance thereof on wearing is lower than that of the tampon which is inserted into the vagina.

Here, with regard to such a kind of interlabial pad, there is not enough development or improvement of a member for discarding.

However, since the interlabial pad is small in size and absorb menstrual blood by the whole of the product unlike the sanitary napkin, it is very difficult to find and pinch a portion which is not stained by menstrual blood to discard it unlike the sanitary napkin. In addition to that, it has significantly low menstrual blood absorbing capacity due to its small size so that menstrual blood absorbed by an interlabial pad may drip down to stain the floor of a bathroom as well as the wearer's fingers while it is removed from the body and thrown to the sanitary trash can. Thus, the disposing process of the pad may be more troublesome compared with other sanitary products.

The present invention intends to achieve the task shown above. It is an object of the present invention to provide an individual wrapping body for an interlabial pad wherein the interlabial pad is wrapped in a wrapping container to realize hygienic and smooth disposal of used interlabial pads.

### Disclosure of the Invention

An individual wrapping body for an interlabial pad according to the present invention has a structure wherein, before unfolding an individual wrapping container, the wrapping container wraps an interlabial pad compactly, and wherein, after unfolding it, it wraps a used interlabial pad completely by making the dimension of the wrapping container larger than before unfolding.

More specifically, the present invention provides the followings.

(1) A wrapping body comprising:
an interlabial pad worn between labia; and
an individual wrapping container for wrapping individually each one of said interlabial pad, in which said interlabial pad is wrapped in said individual wrapping container,
wherein said individual wrapping container is provided with an unwrapping portion for unwrapping,
wherein said container has a shape developable from the unwrapping portion, and
wherein said container has an enough size to enclose a used interlabial pad in a developed state.

According to the present invention, the individual wrapping container (simply referred to as "wrapping container" hereinafter) wrapping an unused interlabial pad can be used for wrapping a used interlabial pad for discarding. Specifically, it is possible to unfold the wrapping container after unwrapping. The dimension thereof gets larger than before unwrapping (the condition that the interlabial pad is wrapped) by unfolding it. So, the used interlabial pad to be discarded can be wrapped completely with the unfolded wrapping material. It prevents the interlabial pad from touching a hand or a garment of a wearer who is performing a discarding process so that menstrual blood absorbed by the interlabial pad does not soil them.

U.S. Patent 6131736 discloses a wrapping body wherein an unused interlabial product is wrapped by a wrapping container that can be re-sealed and the container can be used for discarding a used interlabial product.

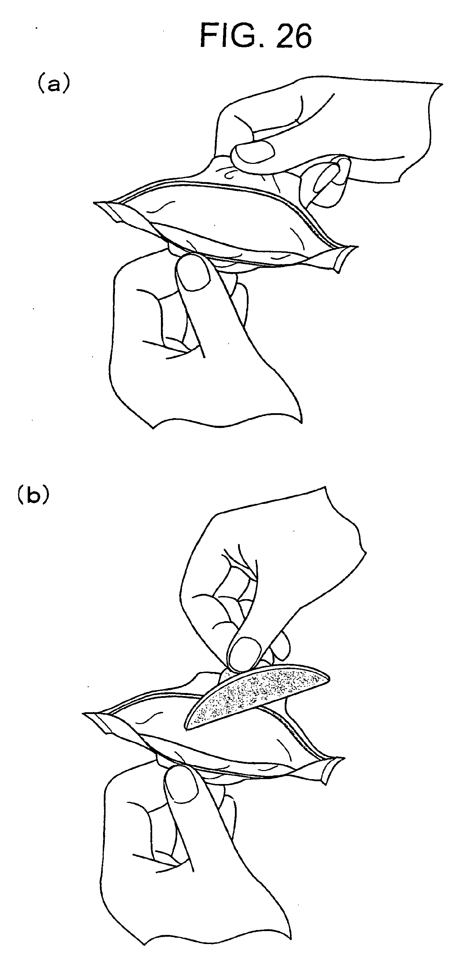

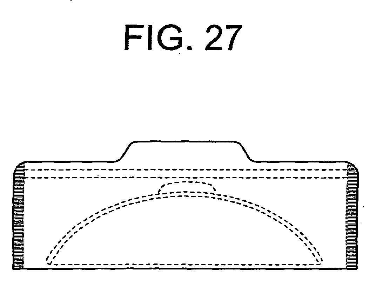

The wrapping body is composed of a wrapping container in which a series of sheet is folded, such that an inside face unfolded along a folding axis thereof and the other inside face unfolded along another folding axis thereof face each other, of which peripheral portions other than the folded parts of the container are sealed, and an in-and-out opening of which is characterized in that at least one sealed side is a re-sealable re-sealing portion to form the opening where the interlabial pad is inserted and withdrawn. An unused interlabial product is retrieved from the wrapping container and an used interlabial product is inserted into the container (refer to Fig. 26). The used interlabial product can be sealed in the wrapping container by re-sealing the re-sealing portion (refer to Fig. 27). Thus, since the used interlabial product is sealed, menstrual blood that absorbed by the interlabial product does not touch a finger so that the used product can be cleanly discarded into a sanitary trash can.

However, in the conventional container, since the wrapping materials are facing each other and since two sides other than the folding parts are completely sealed, the opening area for inserting the used interlabial product is restricted in the opening way. So, in the process of sealing the used product, menstrual blood absorbed by the interlabial product may sometimes adhere to the edge portion of the sealing parts. In this case, the menstrual blood is likely to adhere to a finger, for example, when the sealing inlet opening is re-sealed.

Here, if a wrapping container is designed such that the sealing inlet opening is excessively longer than the dimension of the interlabial product to be contained in the longitudinal or lateral. direction, it is possible to reduce the probability that the trouble shown above occurs. However, such a kind of design makes the wrapping container larger resulting in lack of compactness and portability before unfolding the wrapping container. In addition to that, from the result that the interlabial pad moves too much in the wrapping container by the impact caused during carriage thereofso that the wrapping container may be broken. Thus, an used interlabial product may fall through a broken part after the product has been sealed, or menstrual blood that has been absorbed in the interlabial pad may leak out and adhere to a hand, etc.

In this respect, according to the present invention, when the used interlabial pad is discarded, the apparent size (area) thereof can be made larger than before retrieving the unused interlabial pad by developing the wrapping container after retrieving the unused interlabial pad. That is, the apparent size could be made larger than the wrapping body containing the interlabial pad. By this, when wrapping the used interlabial pad with the wrapping material developed from the wrapping container, a hand holding the wrapping material can be covered so as to prevent the absorbed menstrual blood from adhering to the hand without making the wrapping container excessively larger.

As this, in the wrapping body according to the present invention, since the size of the material to wrap an interlabial pad changes before and after unfolding the wrapping container, the used interlabial pad can be sealed securely without deteriorating the compactness of the original shape before unfolding. In addition to that, since the used interlabial pad is sealed in the wrapping material, the wrapping container enables the discarding process to be hygienic and clean in appearance.

In the present invention, the wrapping container is preferably unfolded into the wrapping material that has an area being at least twice as large as before unfolding in order to completely cover a hand holding the wrapping material.

(2) The wrapping body according to (1),
wherein said individual wrapping container comprises a series of wrapping sheet superposing each other, the sheets comprising two ends and being wrapped in a way to form an overlapping part where the both ends of the wrapping sheet and a vicinity of the both ends are overlapped each other,
wherein said unwrapping portion is formed to unwrap said individual wrapping container by releasing an overlapping state of the overlapping part; and
wherein said wrapping sheet turns to be a waste discarding sheet for enclosing a used interlabial pad in a state that said wrapping container is developed after unwrapping said individual wrapping container.

According to the present invention, the individual wrapping container (referred to simply as "wrapping container" hereafter) is formed by folding a sheet in such a way that it rolls up an interlabial pad. The wrapping container can be used as a discarding material to wrap the used interlabial pad completely by unfolding the folded area so as to make the wrapping container into a flat sheet. So, an unused interlabial pad is wrapped compactly with the folded sheet before unsealing and an used interlabial pad can be wrapped completely with the unfolded flat sheet after unsealing.

According to the present invention, the whole wrapping container may be unfolded to make a flat state in order to remove an unused interlabial pad. Or a part of the wrapping container may be unfolded to make a flat state in order to remove an unused interlabial pad. Here, if only a part of the wrapping container is unfolded, in order to unfold the whole wrapping body when an used interlabial pad is wrapped, for example, it is also possible to provide an area where a part of sheet is folded near the unsealing part such that only the folded part is unfolded and fattened in an expanding way to wrap the used interlabial pad.

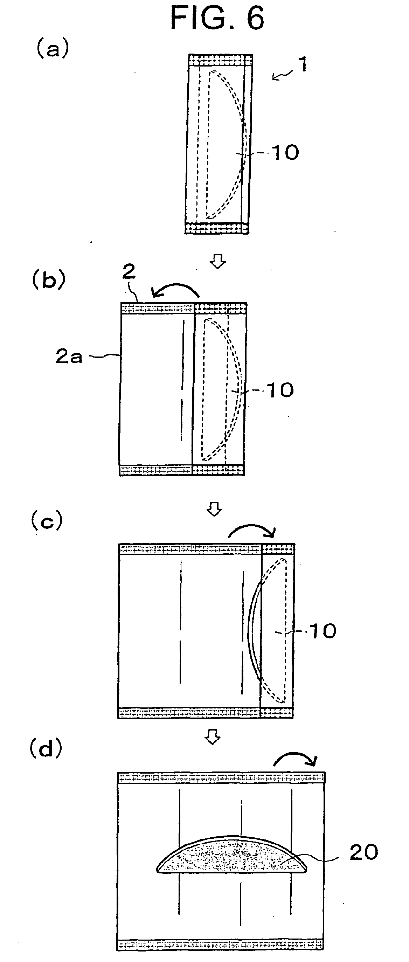

In addition to that, according to the present invention, since the wrapping container is unfolded to a flat sheet, it is not required to contain an used interlabial pad in the same direction as to contain an unused interlabial pad. For example, when the wrapping container with the folded sheet is unfolded in the lateral direction of the interlabial pad that has an elongated shape, by making the maximum unfolding width be larger than the longitudinal dimension of the interlabial pad, the wrapping sheet may have the dimension large enough to cover the whole interlabial pad even if the interlabial pad is aligned in a different direction before unfolding from after unfolding (refer to Fig. 6).

As described above, according to the present invention, unlike a conventional example, an used interlabial pad may be contained not necessarily in the same condition as an unused interlabial pad. Moreover, since the wrapping container can be unfolded flatly, there is no burden that the used interlabial pad should be contained carefully so that menstrual blood does not adhere to the vicinity of the inlet of the wrapping container as a conventional example.

Further, since the unsealing direction when retrieving an unused interlabial pad from the wrapping container is aligned to the direction to unfold the wrapping container so as to seal a used interlabial pad, it is unlikely that a wearer is confused under a condition that she should replace the product in haste and that replacing work is more simplified.

The front and rear ends of the sheet used for the wrapping material are bonded in such a way that a wearer can peel them on unsealing. They are bonded to each other to be re-unsealable with techniques such as pressure by heat emboss, interlace by male and female emboss portions as known in the art.

(3) The wrapping body according to (2), wherein said wrapping sheet has fine protrusions on a surface of at least one face of said wrapping sheet.

According to the present invention, minute bosses or projections are formed on at least one surface of a series of sheet constituting the individual wrapping container (referred to simply as "wrapping container" hereafter). So, menstrual blood flown from the interlabial pad does not adhere to the sheets so as to make the sheets tightly contact with each other, but is kept between the sheets with a small distance from surfaces of each other by the minute bosses on the surfaces. As a result, an incident that menstrual blood is observed significantly from outside of the sheet can be evaded to realize cleaner appearance compared to the case where the interlabial pad is wrapped in a sheet without any bosses.

(4) The wrapping body according to (2) or (3), wherein said wrapping sheet comprises an inner face to be in contact with the interlabial pad to be wrapped and an outer face to be held by the wearer, the wrapping body comprising
a bent portion to a side of said inner face provided in a vicinity of one end of the two ends of said wrapping sheet in the longitudinal direction,
wherein said bent portion forms a pocket portion capable of receiving said used interlabial pad.

According to the present invention, the individual wrapping container (referred to simply as "wrapping container" hereafter) is composed of a series of sheet, and further, a pocket portion with a size that an interlabial pad can be contained with a folded part of the sheet. So, a wearer can put an used interlabial pad into the pocket section when the wrapping container is used as a discarding member after removing an unused interlabial pad.

In addition to that, since all portions other than the pocket portion can be flattened after unfolding the wrapping container, it is possible to cover the inlet of the pocket portion using the flat part to seal the interlabial pad in the pocket portion. Thus, sealed condition of the interlabial pad is maintained until it is discarded completely. And since the opening of the pocket portion can be covered completely, even if the used interlabial pad contacts the vicinity of the opening and menstrual blood absorbed in the interlabial pad when containing the used interlabial pad in the pocket portion, the menstrual blood does not adhere to a wearer's hand so as to secure hygienic disposal.

According to the present invention, the wrapping container may be formed by folding one end of a series of sheet, which has a shape elongated in the longitudinal direction, towards the face (inner surface) on which the interlabial pad is placed and by bonding both side edges or one side edge of the wrapping sheet around the overlapping part of the wrapping sheet so as to make the pocket portion. It is preferable that both side edges at the front and back of the pocket portion are not peeled off in order to secure the bonding portion of the wrapping container so that the pocket portion is not broken when the used interlabial pad is put into the pocket portion. In order to prevent peeling off, male and female emboss rates may be raised to deepen the engagement between the wrapping materials, or the wrapping materials may be bonded by heat fusion with an increased temperature during the emboss process, or an adhesive may be applied between the wrapping materials.

(5) The wrapping body according to (4), wherein said wrapping sheet is folded in a way to have one or more folded portions where a part of said wrapping sheet is bent toward a side of said inner face near an end opposite to the end where said pocket portion is provided;
wherein said used interlabial pad can be wrapped with said waste discarding sheet including, as a part of said waste discarding sheet, said folded portions that are developed in a state after said individual wrapping container is unwrapped and developed; and
wherein said pocket portion can receive said used interlabial pad that is wrapped with said part of said waste discarding sheet.

According to the present invention, in the individual wrapping container (referred to simply as "wrapping container" hereafter) after removing an interlabial pad when an used interlabial pad is discarded, it is possible to unfold the folded portion locating at one end of the wrapping sheet constituting the said wrapping container so as to enlarge the flat area and wrap the interlabial pad with the unfolded and extended area, and it is also possible to put the used interlabial pad, with the above-state unchanged, in the pocket portion formed in the other end of the wrapping sheet. This prevents menstrual blood absorbed in the interlabial pad from oozing and the interlabial pad from protruding out of the end of the wrapping sheet.

In order to place the used interlabial pad in the pocket portion, the surface of the interlabial pad, which has contacted the body and absorbed menstrual blood, is made to touch said extended area and slide into the pocket portion. This operation prevents menstrual blood from soiling the edge of the inlet opening of the pocket portion resulting in hygienic and smooth placement of the interlabial pad in the pocket portion.

(6) The wrapping body according to (4) or (5), wherein a dimension of an opening of said pocket is longer than a dimension of a bottom of said pocket.

According to the present invention, the opening to be the inlet opening of the pocket portion with the size (or dimension) as the interlabial pad can be contained is larger than the dimension of the bottom of the pocket portion. So, the size of the inlet opening is larger than that of the interlabial pad so that the used interlabial pad may be placed into the pocket portion smoothly.

In order to increase the length dimension around the inlet opening of the pocket portion, methods such as making the cut edge of the inlet opening curved, sinuate, oblique, etc. can be adopted . According to the present invention, since the pocket portion is formed by folding a series of sheet, the cut described above is applied at one end forming the pocket sheet.

(7) A wrapping body comprising:
an interlabial pad worn between labia; and
an individual wrapping container for wrapping individually each one of said interlabial pad, wherein the wrapping body comprises said individual wrapping container wrapping said interlabial pad,
wherein said individual wrapping container is provided with an unwrapping portion for unwrapping, and a shape developable from the unwrapping portion; and
wherein said individual wrapping container comprises an elongated area; and
wherein said individual wrapping container turns to be of a size to enclose a used interlabial pad in a developed state by extending said elongated area.

According to the present invention, the sheet constituting the wrapping container is itself composed of elastic material. So, it is possible to make the dimension of the sheet larger by extending the sheet when a used interlabial pad is wrapped. The sheet, however, has a compact size before wrapping the used interlabial pad.

Here, generally, when an elastic material is extended, in the dimension in the direction perpendicular to the extended direction, the necking (dimensional contraction in) of the elastic material occurs to make said dimension smaller than before extension. Thus, when wrapping a used interlabial pad, there is a possibility that the whole interlabial pad may not be wrapped completely depending on the alignment of the interlabial pad.

Consequently, the dimension that is perpendicular to the direction of extension of the wrapping material is preferably at least the same as or longer than the dimension of the longest part of the interlabial pad.

According to the present invention, since the material to constitute the wrapping container may be extended from the original size in the phase of wrapping the used interlabial pad, they may have either retractility or extensity. If the material has retractility, it is extended when covering the used interlabial pad with the wrapping material and retracted after wrapping it. The used interlabial pad can be wrapped more compactly. As a result, the wasted wrapping body becomes compact so as to prevent a sanitary trash can from being filled soon.

(8) The wrapping body according to (7), wherein said elongated area is formed by an emboss processing.

According to the present invention, the sheet constituting the wrapping container may be processed with an embossing process. So, when a used interlabial pad is wrapped with theunfolded wrapping container, the sheet is extended from an ordinary state because the concavity and convexity obtained with the emboss processing are flattened.

(9) The wrapping body according to any one from (1) to (8), wherein said unwrapping portion of said individual wrapping container is provided with a sealing means that is re-sealable.

According to the present invention, a re-sealable sealing means is equipped on the unwrapping portion of the wrapping container. So, when a used interlabial pad is enclosed with the wrapping material after unfolding the wrapping container, the interlabial pad can be sealed with said sealing means securely so that it may be prevented that the sealed state is released before discarding the used interlabial pad sealed with the wrapping material such that the interlabial pad protrudes out of the wrapping container, and that menstrual blood adheres to the pad may stain a hand, and that the used interlabial pad alone may fall on the floor, and so on. Therefore, a hygienic disposal process may be secured more securely by reducing occurrence of such incidents described above.

For the re-sealable sealing means, publicly known technologies such as a film layer with an adhesive on one side, a tissue with an adhesive on one side considering flushability, and so on may be employed. Other sealing means may include MAGIC TAPE (Japanese registered trademark) fastening (like Velcro (registered trademark)), a zipper to interlace male and female parts, and so on.

(10) The wrapping body according to any one from (1) to (9),
wherein said interlabial pad is contained in a bent state before said individual wrapping container is developed; and
wherein said used interlabial pad is contained in a developed state when said individual wrapping container is developed to enclose said used interlabial pad.

According to the present invention, an unused interlabial pad is folded and contained in the individual wrapping container (referred to simply as "wrapping container" hereafter) so that the wrapping body may be compact when it is carried. However, when the wrapping body is unwrapped and when an used interlabial pad is discarded, since the wrapping container is unfolded, the used interlabial pad can be wrapped as it is without folding it into the original shape of the unused interlabial pad. So, the wearer may obtain double merits such as portability of the wrapping body to be carried for spare and hygienic disposition of the used interlabial pad having absorbed menstrual blood.

(11) The wrapping body according to any one from (1) to (10),
wherein said interlabial pad is provided with a finger insertion opening,
in which a finger can be inserted, on an opposite side face to a body side face; and
wherein said finger insertion opening is disposed near said unwrapping portion of said individual wrapping container, and
wherein the finger insertion opening of said contained interlabial pad can be exposed naturally at a beginning of unwrapping the individual wrapping container when said individual wrapping container is unwrapped.

According to the present invention, the interlabial pad in the wrapping body has a finger insertion opening by which the interlabial pad is held at a fingertip, and the finger insertion opening of the interlabial pad can be observed through the unwrapping portion from outside of the wrapping container when it is unwrapped, and a finger can be inserted through the unwrapping portion into the finger insertion opening. So, it is possible to wear an unused interlabial pad quickly and discard a used interlabial pad quickly so that the time for the total process to replace the interlabial pad may be shortened.

### Brief Description of the Drawings

Fig. 1 is a perspective view indicating the first embodiment of the wrapping body according to the present invention.
Fig. 2 is a cross section A-A of the wrapping body in Fig. 1.
Fig. 3 is a perspective view indicating the first embodiment of the wrapping body containing the interlabial pad folded along the longitudinal direction as a central axis.
Fig. 4 is a cross section B-B of the wrapping body in Fig. 3.
Fig. 5 is an extend elevation in a cross sectional view of the first embodiment of the wrapping body.
Fig. 6 is an extend elevation seen from the above according to the first embodiment of the wrapping body.
Fig. 7 is a graphical representation indicating the wrapping body equipped with a folding area in the longitudinal direction.
Fig. 8 is an extend elevation of the wrapping body in Fig. 7.
Fig. 9 is a process chart of discarding the used interlabial pad.
Fig. 10 is a longitudinal sectional view of the second embodiment of the wrapping body composed of the wrapping container having a pocket portion.
Fig. 11 is a process chart showing the process from unfolding the wrapping container of the wrapping body according to the second embodiment to removing an unused interlabial pad.
Fig. 12 is a process chart showing the process from unfolding the wrapping container of the wrapping body according to the second embodiment to putting an used interlabial pad into the pocket portion and covering the inlet thereof.
Fig. 13 is a graphical representation showing the wrapping container where the opening of the pocket portion of the wrapping container according to the second embodiment is curved.
Fig. 14 is a graphical representation showing the wrapping container where an adhesive is applied to the pocket portion of the wrapping container according to the second embodiment.
Fig. 15 is a graphical representation showing the wrapping body where an adhesive is applied on the pocket portion of the wrapping container according to the second embodiment in order to enhance the sealing state.
Fig. 16 is a longitudinal sectional view of the wrapping body according to the third embodiment, where the pocket portion and the folding area are provided at the unsealing opening.
Fig. 17 is a view of a process of unfolding the wrapping container of the wrapping body according to the third embodiment.
Fig. 18 is a graphical representation of a process of wrapping a used interlabial pad in the unfolded sheet of the wrapping container of the wrapping body according to the third embodiment.
Fig. 19 is a schematic diagram showing the state before and after extending the sheet constituting the wrapping container according to the fourth embodiment.
Fig. 20 is a schematic diagram showing the state that an used interlabial pad is wrapped in the sheet constituting the wrapping container of the fourth embodiment.
Fig. 21 is a graphical illustration of an embodiment of embossing process applied to the sheet.
Fig. 22 is a graphical illustration of an embodiment of embossing process applied to the sheet.
Fig. 23 is a longitudinal sectional view showing another embodiment of the unsealing section of the wrapping container where the wrapping container is constituted by the substantial two folded sheet.
Fig. 24 is a longitudinal sectional view showing another embodiment of the unsealing section of the wrapping container where a fixing tape is provided at the unsealing section of the wrapping body.
Fig. 25 is a longitudinal sectional view showing the wrapping body containing an interlabial pad with a mini sheet piece.
Fig. 26 is a graphical drawing showing the state where an used interlabial product is to be contained in a conventional wrapping container.
Fig. 27 is a graphical drawing showing the state that an used interlabial product is contained in a conventional wrapping container.

### Best Mode of Carrying Out the Invention

The embodiments according to the present invention will now be explained with figures below.

### [First embodiment]

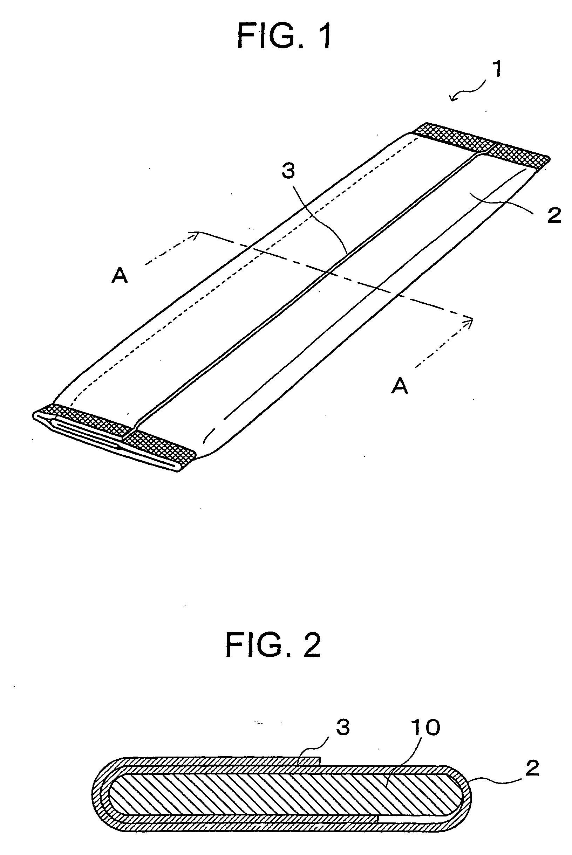

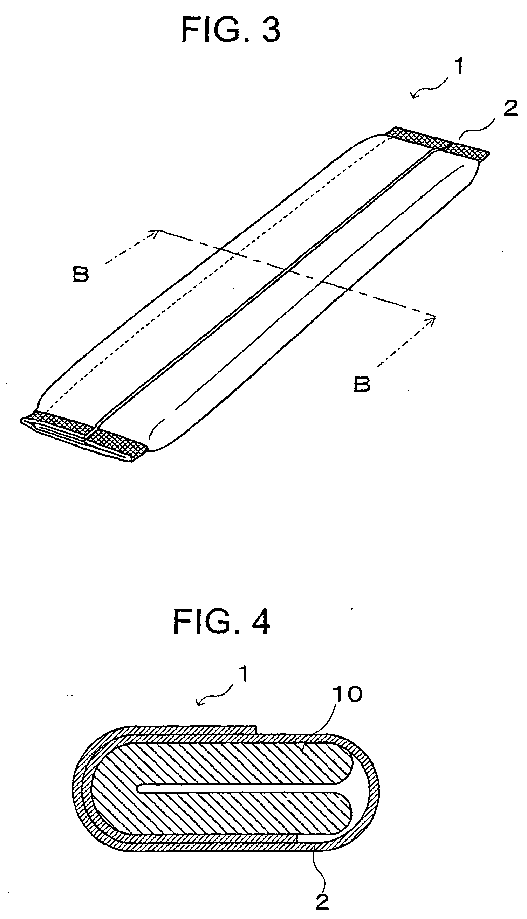

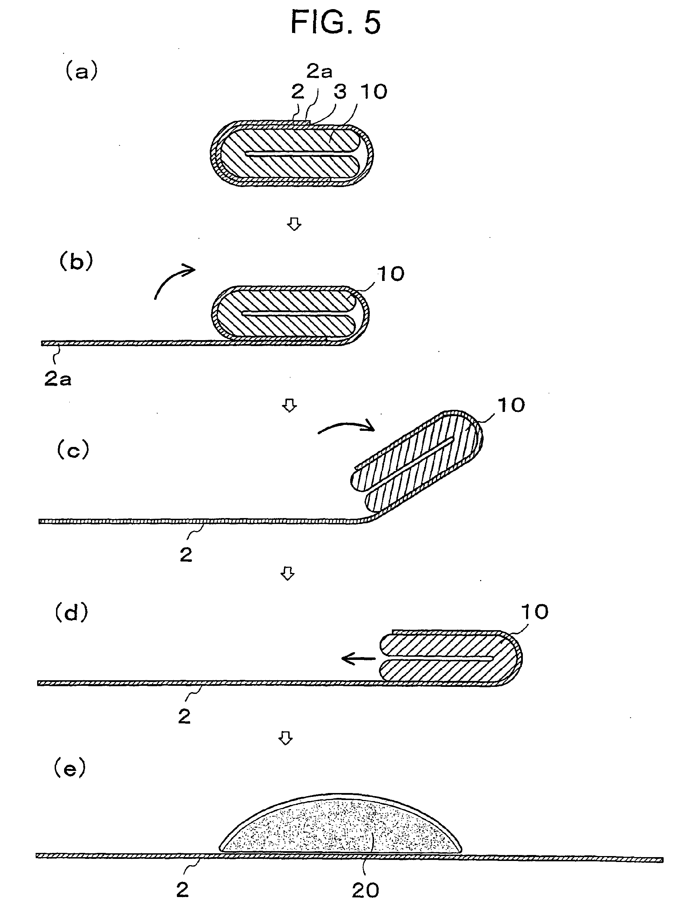

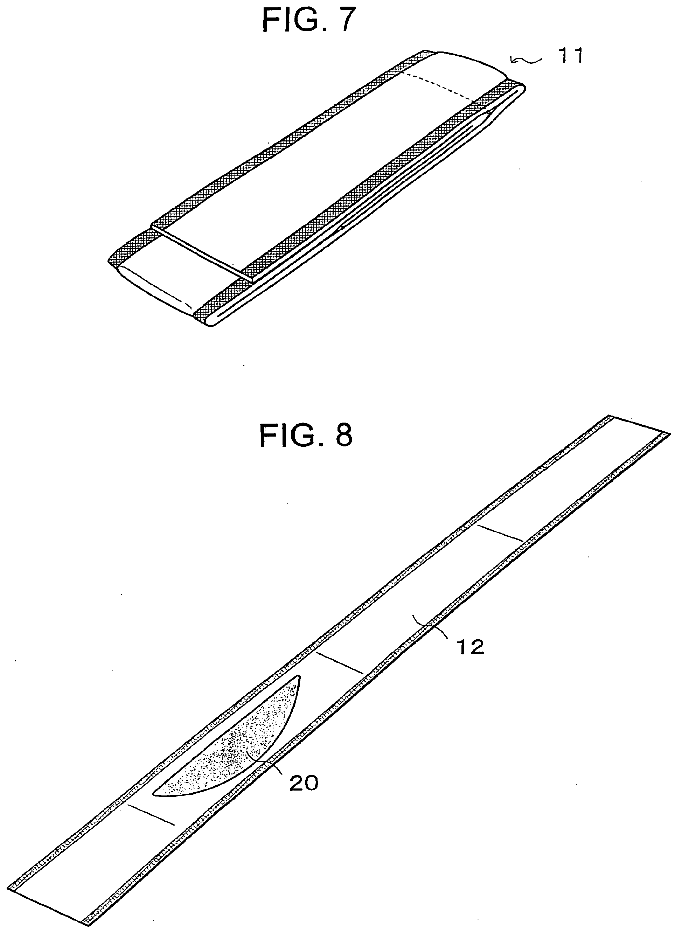

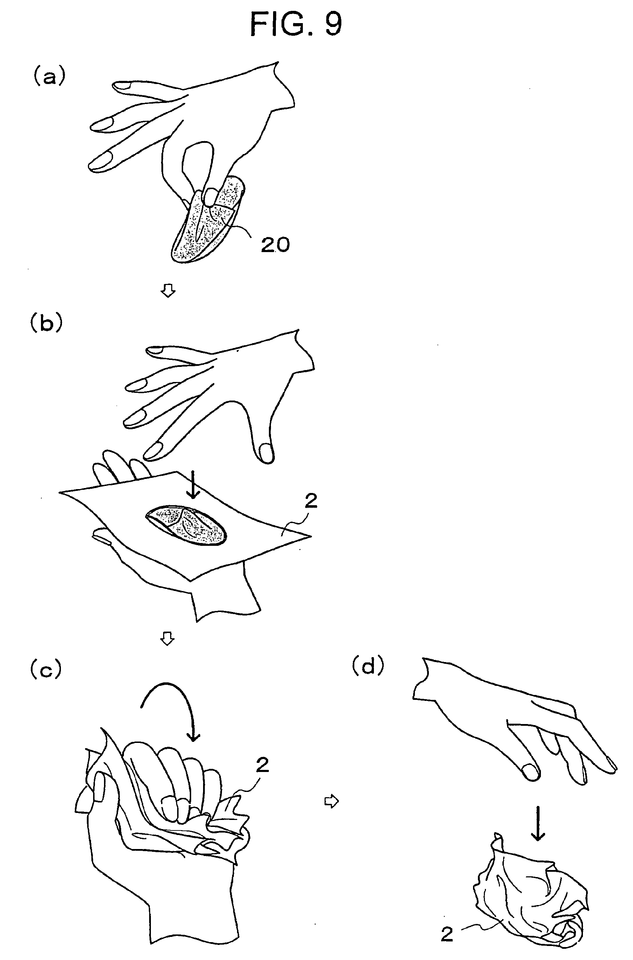

The structure of the wrapping body according to the first embodiment will now be explained. Fig. 1 is an perspective view indicating the first embodiment of the wrapping body. Fig. 2 is a cross section A-A of the wrapping body in Fig. 1. Fig. 3 is an perspective view indicating the wrapping body 1 containing the interlabial pad 10 folded. Fig. 4 is a cross section B-B of the wrapping body 1 in Fig. 3. Fig. 5 is an extend elevation of a cross section of the wrapping body 1 in Fig. 4. Fig. 6 is an extend elevation seen from above the wrapping body 1 in Fig. 3. Fig. 7 is a graphical representation indicating the wrapping body 11 provided with a folding area in the longitudinal direction. Fig. 8 is an extend elevation of the wrapping body 11 in Fig. 7. Fig. 9 is a process chart of discarding an used interlabial pad 20.

As shown in Fig. 1, the wrapping body 1 is composed of the wrapping container constituted of a series of sheet 2 and contains an unused interlabial pad 10 in such a manner that it wraps the pad with sheet 2 as shown in Fig. 2.

The upper and lower ends of the wrapping container are bonded in re-sealable fashion so that they do not overlap the interlabial pad 10 contained. The unsealing section 3 is formed at the section where the sheet 2 are overlapped. The unsealing section 3 is also bonded in re-sealable fashion. An adhesive may be used with heat sealing or male and female embossment known in the art in order to make the bonding state stronger. Adhesives may be selected from the hot melt type adhesives without particular limitation. They are applied in the patterns such as sheet pattern, banded pattern, spiral pattern, O pattern as known in the art.

In this embodiment, the interlabial pad 10 contained can be folded in half along the longitudinal direction as the center axis. In this case, the wrapping body 1 shown in Fig. 3 becomes narrower in width than that shown in Fig. 1 resulting in further compactness when taken along. The interlabial pad 10 to be contained is wrapped in the sheet 2 in the manner shown in Fig. 4.

The sheet 2 of the wrapping body 1 is folded in the lateral direction. So, when opening the wrapping body 1, first, pinch the one end 2a of the sheet 2 constituting the sealing section 3 that is sealed in re-sealable fashion in the wrapping body 1 shown in Fig. 5 (a), open it outwards as shown Fig. 5 (b), then, grip the sheet 2 with the interlabial pad 10 in whole as shown in Feb. 5 (c) and open it outwards to expose the interlabial pad 10 as shown in Fig. 5 (d) and remove it to use. As described above, after taking out the interlabial pad 10, the used interlabial pad 20 can be wrapped completely in the sheet 2 even if the orientation of the used interlabia pad is different from that of the unused interlabial pad 10 since the wrapping container can be unfolded completely as shown in Fig. 5 (e).

The unwrapping process of wrapping body 1 will now be explained in more detail. The wrapping container constituting the wrapping body 1 that is compact before unsealing as shown in Fig. 6 (a) is unfolded into the sheet 2 in planate shape through the process of Fig. 6 (b) and (c). As a result, the wrapping container is unfolded into a discarding member having an area larger than the wrapping body 1 shown in Fig. 6 (a). By this, as shown in Fig. 6 (d), the used interlabial pad 20 can be wrapped even if it is different from the unused interlabial pad 10 in the orientation.

In this respect, as the wrapping body 11 shown in Fig. 7, if the sheet 12 is folded in such a manner that folding area is provided in the longitudinal direction of the wrapping container, the wrapping container is folded into the shape as shown in Fig. 8 when the wrapping body 11 is unwrapped. If the used longitudinal interlabial pad 20 is wrapped in the sheet 12 shown above, the areas on left and right sides that can not be sealed are enlarged. So, the used interlabial pad should be wrapped in the sheet 12 in the same orientation as the unused interlabial pad 10. In addition to that, the used interlabial pad 20 is highly likely to protrude from the left and right sides of the sheet 12 resulting in adhesion of menstrual blood onto the hands of a wearer when the used interlabial pad 20 covered by the sheet 12 into a sanitary container. On the other hand, the wrapping body 1 of the embodiment is unfolded in such a manner that the wrapping container has enough size and shape to wrap the interlabial pad 20 and no trouble as shown above occurs.

The method to discard an used interlabial pad 20 is that, pinch the used interlabial pad 20 removed from the interlabial space as shown in Fig. 9 (a), receive the interlabial pad 20 with the hand covered by the sheet 2 that is unfolded from the wrapping container as shown in Fig. 9 (b), wrap the entire interlabial pad 20 in the sheet 2 securely as shown in Fig. 9 (c) and discard it into a sanitary container as it is as shown in Fig. 9 (d).

### [Second embodiment]

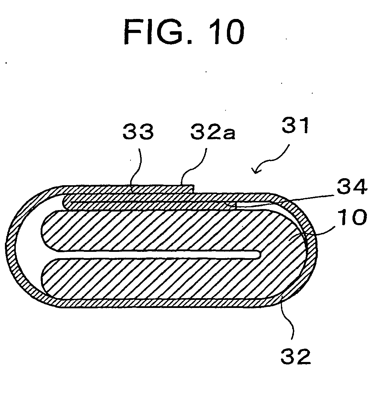

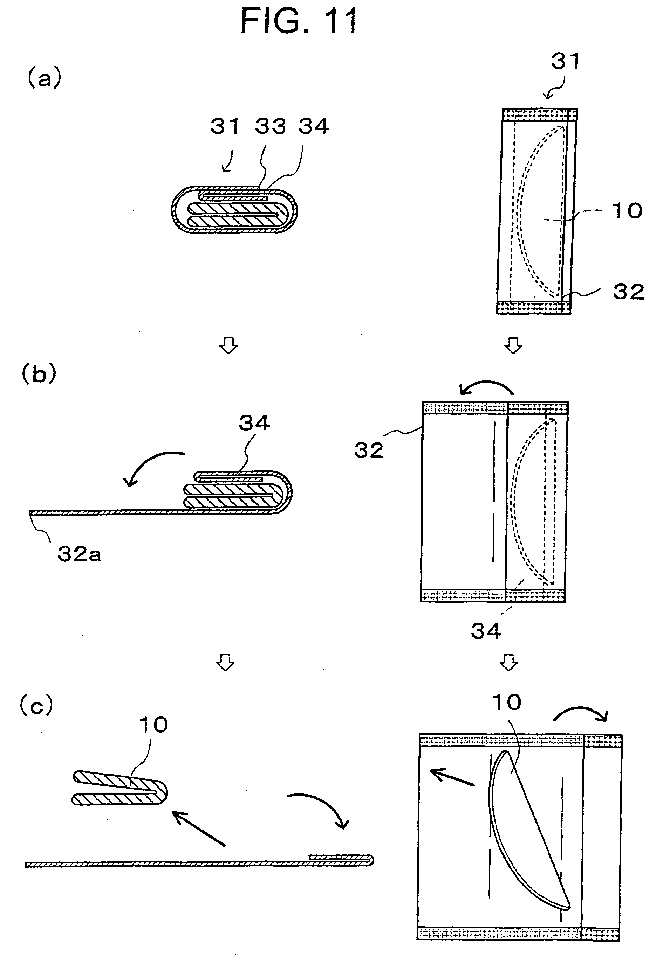

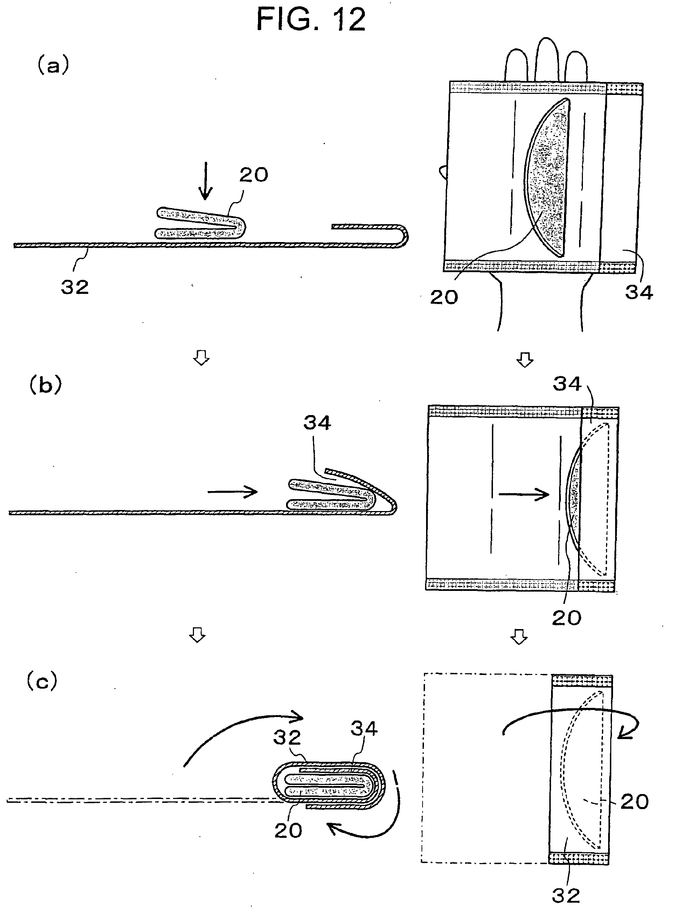

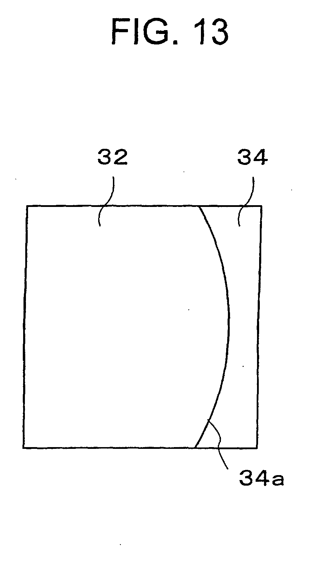

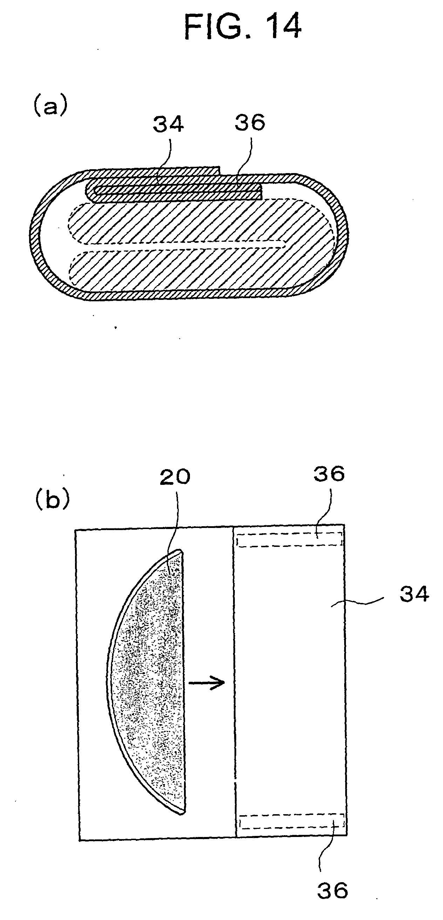

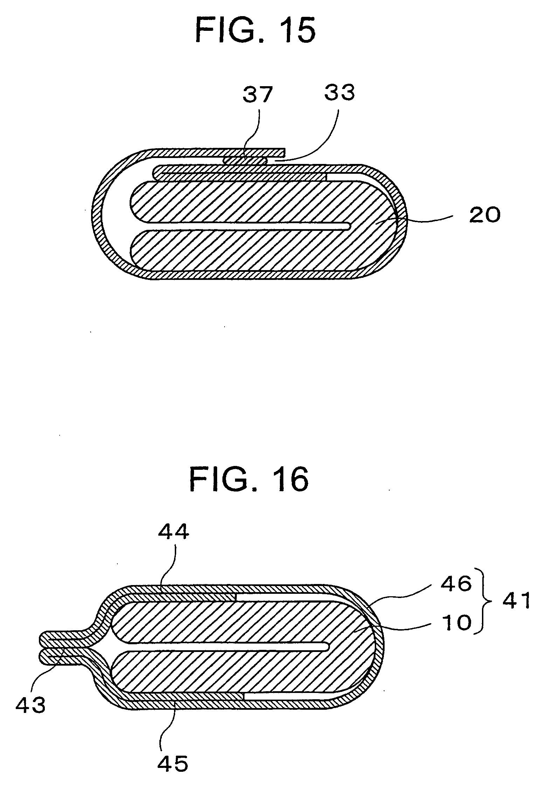

The second embodiment of the wrapping body constituted of the wrapping container with a pocket portion will now be explained. Fig. 10 is a longitudinal sectional view of the wrapping body composed of the wrapping container 31 having the pocket portion 34. Fig. 11 is a process chart showing the process to remove an unused interlabial pad 12 by unfolding the wrapping body 31. Fig. 12 is a process chart showing the process to put an used interlabial pad 20 into the pocket portion 34 and cover the inlet thereof. Fig. 13 is a graphical representation showing the wrapping container where the opening of the pocket portion 34 is curved. Fig. 14 is a graphical representation showing the wrapping container where an adhesive 36 is applied to the pocket portion 34. Fig. 15 is a graphical representation showing the wrapping container where an adhesive 37 is applied on the pocket portion 34 resulting in enhanced sealing state.

### <Basic structure>

As shown in Fig. 10, in the wrapping body 31, the one side of the rectangular shaped sheet 32 in the longitudinal direction is turned in independently of wrapping the interlabial pad 10. The pocket portion 34 is formed at the interfolded section. The unsealing section 33 to open the wrapping container is formed by folding the sheet 32 in such a manner that the other end 32a where the pocket portion 34 is not formed is overlapped on outside of the pocket portion 34, and bonding the overlapped section in re-sealable fashion with press force by heat embossing, etc. and interlacing by male and female embossing known in the art. Upper and lower end of the wrapping container are bonded in re-sealable manner in such a way the interlabial pad 10 contained is not overlapped.

### <Unsealing state>

The wrapping body 31 compactly containing the interlabial pad 10 as shown in Fig. 11 (a) is unwrapped by pinching the one end 32a of the sheet 32 to open the unsealing section 33 as shown in Fig. 11 (b) and the interlabial pad is exposed and taken out as shown in Fig. 11 (c).

Then, as shown in Fig. 12 (a), the used interlabial pad 20 is put on the sheet 32 after the wrapping container is unfolded with the body-facing side contacted the sheet 32, slid into the pocket portion 34 as shown in Fig. 12 (b), finally the outside of the pocket portion 34 is wrapped in the unfolded section of the sheet 32 as shown in Fig. 12 (c) to seal the opening of the pocket portion 34, and the interlabial pad 20 is securely enclosed in the pocket portion 34. The used interlabial pad is discarded into the sanitary container in this state.

As shown in Fig. 13, the opening 34a of the pocket portion 34 can be curved. This opening 34a is the sealing inlet of the pocket portion 34 of the used interlabial pad 20. So, the circumference of the sealing inlet can be enlarged by cutting it curvedly. By this, the interlabial pad 20 can be slid and contained in the pocket smoothly. In addition, the risk that adhesion of menstrual blood near the sealing inlet can be reduced.

As shown in Fig. 14 (a), it is possible to apply the adhesion 36 on the both ends of the pocket portion 34. By this, as shown in Fig. 14 (b), it is possible to prevent the pocket portion 34 from being broken in the process of sealing the used interlabial pad 20.

Further, as shown in Fig. 15, it is also possible to apply the adhesive 37 on a part of outside of the pocket portion 34. By this, it is possible to fortify the sealing state of the unsealing section 33 after wrapping the used interlabial pad 20.

### [Third embodiment]

The third embodiment of the wrapping body constituted by the wrapping container provided with the pocket portion and the folding section at the unsealing opening.

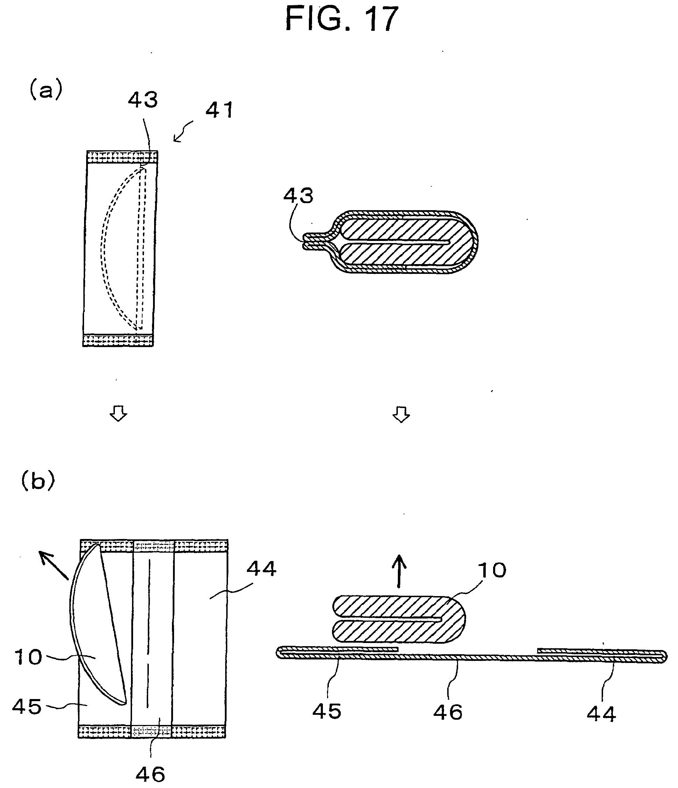

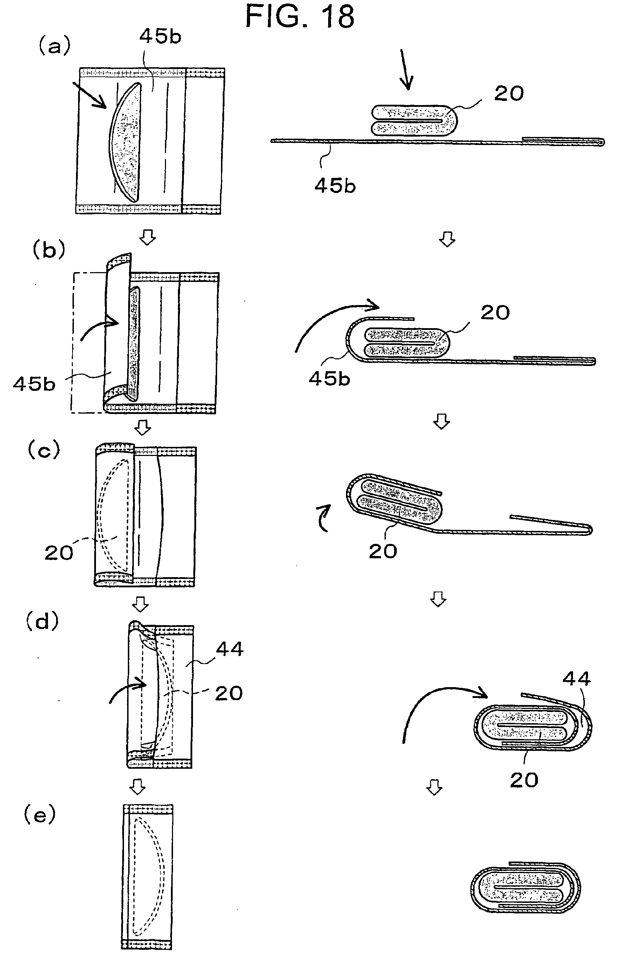

Fig. 16 is a longitudinal sectional view of the wrapping body 41. Fig. 17 is a graphical illustration of the process of unfolding the wrapping container of the wrapping body 41. Fig. 18 is a graphical illustration of the process of wrapping the used interlabial pad 20 in the sheet 42.

As shown in Fig. 16, the wrapping body 41 comprises the wrapping container 46 containing the interlabial pad 10, provided with the pocket portion 44 and the folding section 45 to be extendable after unsealing near the unsealing section 43.

In this kind of the wrapping body 41, the interlabial pad 10 contained as shown in Fig. 17(b) is taken out by opening the unsealing section 43 shown in Fig. 17(a) to unseal the wrapping container 46.

Subsequently, the extended area 45b is obtained by opening the folding section 45 to the left side. The used interlabial pad 20 is put on the extended area 45b as shown Fig. 18(a). Then, the interlabial pad 20 is wrapped by the extended area 45b as shown in Fig. 18(b) in such a way that the interlabial pad can not be seen as shown in Fig. 18(c). With this state, the interlabial pad 20 is inserted into the pocket portion 44 as shown in Fig. 18(d) to make a compact state as shown in Fig. 18(e). By this, it is possible to prevent the wrapping state of the interlabial pad 20 from deforming until the used interlabial pad is discarded so as to achieve clean discard of the used interlabial pad 20.

### [Fourth embodiment]

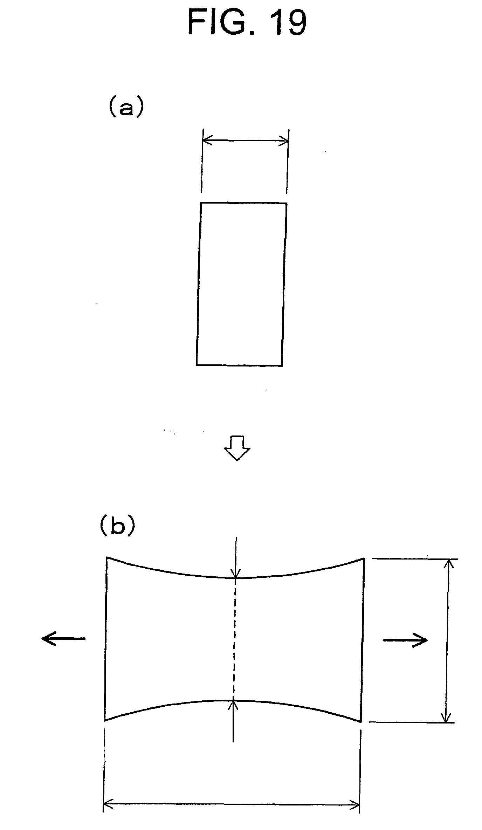

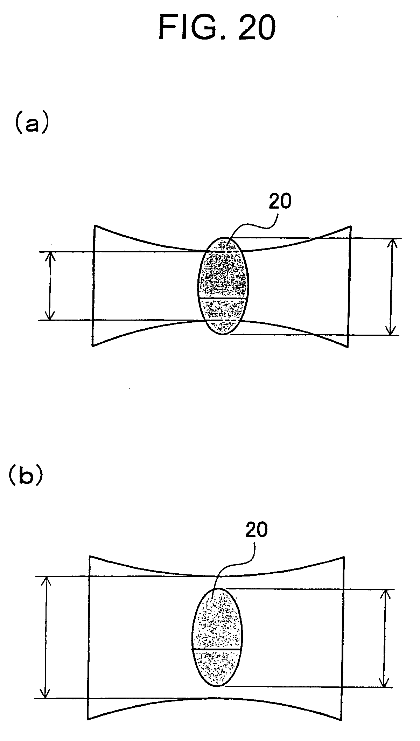

Stretch sheet materials that are usable in the wrapping container will now be explained. Fig. 19 is a graphical illustration to compare the states of the sheet before and after extension. Fig. 20 is a schematic diagram to explain the state that the used interlabial pad is wrapped.

In the present invention, the dimension of the wrapping material to wrap an unused interlabial pad may be enlarged when wrapping the unused interlabial pad. The wrapping material that can be extended depending on an intention of a wearer when required can be used for the wrapping container. However, as shown in Fig. 19, if a material with extensity is extended in the lateral direction, necking, a contraction in width (the distance between upper and lower end is reduced), usually occurs in the middle of the material. So, even if the length before extension is larger than that of the interlabial pad in the longitudinal direction, the interlabial pad 20 may protrude when the material is extended as shown in Fig. 20(a). Thus, it is required to secure an enough length that the interlabial pad 20 is wrapped completely when the material is extended as shown in Fig. 20(b).

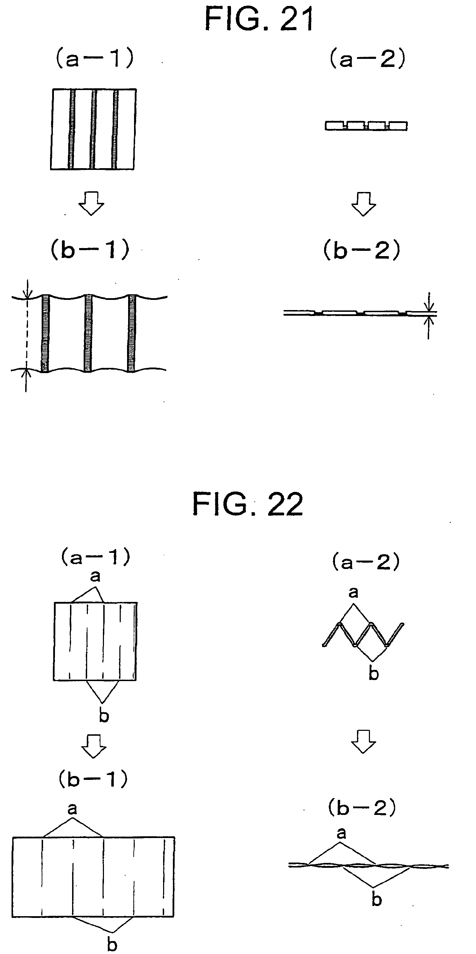

There are methods for embossing in order to reduce occurrence of necking area in stretch materials. For example, the occurrence of the necking area can be reduced by applying embossing process by passing the wrapping sheet between two rollers having a patterned indented surface and a flat surface, respectively. In this case, as shown in Fig. 21(a-1)and(a-2), only the concave areas have higher density. When extended, thickness of the sheet is reduced due to decreased height of the flat areas. Thus, dimensions of entire sheet can be enlarged with area of neck-in reduced.

The occurrence of necking area can be reduced without losing extensity of the wrapping sheet by passing it between two engaging rollers having patterned indented surfaces. In this case, as shown in Fig. 22 (a-1)and (a-2), mountains a and valleys b having high density are formed by turns. When extended, areas other than the mountains a and valleys b are stretched to make the dimension of entire sheet larger. As a result, there is little neck-in area as shown in Fig. 22 (b-1).

### [Other modes of the unsealing section]

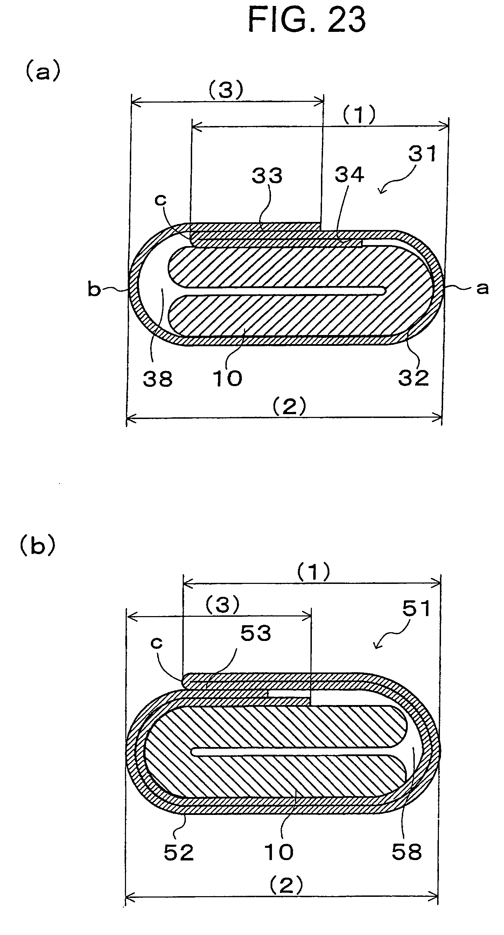

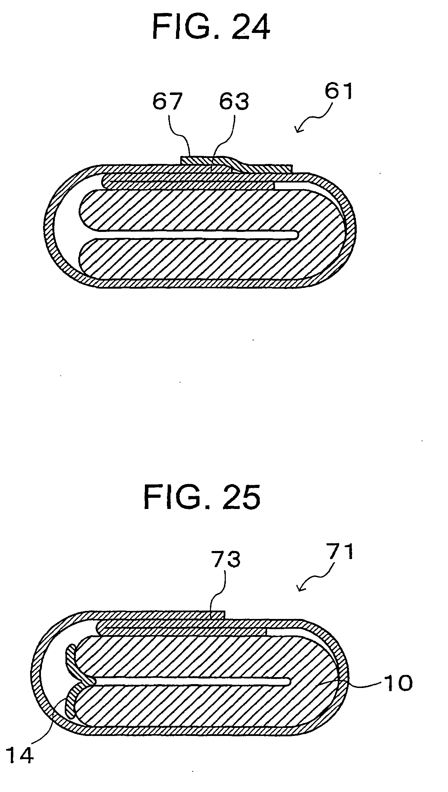

Other modes of the unsealing section of the wrapping container will now be explained. Fig. 23 is a longitudinal sectional view of the wrapping body 51 where the wrapping container is constituted by the wrapping body 31 shown in Fig. 10 and sheet 52 substantially folded in half. Fig. 24 is a graphical illustration of the wrapping body 61 where a fixing tape 67 is attached to the unsealing section.

As shown in Fig. 23 (a), the wrapping body 31 can be formed by folding the sheet 32 that is to be the wrapping material along the first folding line a that is folded back from the first surface to the second surface and the second folding line b that is folded back from the second surface to the third surface in such a manner the wrapping body has the first (1), second (2) and third (3) surface and covering the interlabial pad 10 along the lateral direction with the wrapping sheet 32. In this case, the wrapping container is constituted of the first surface facing the upper surface of the interlabial pad 10, the second surface provided on the lower surface covering the folding section of the interlabial pad 10 and the third surface folded back towards the upper surface. By overlapping the first surface and the third surface, the unsealing section 33 is formed at the one side edge opposite to the second folding line b of the third surface and the takeoff opening 38 of the interlabial pad 10 is formed at the one side edge opposite to the first folding line a of the first surface.

Here, the folding section is formed inside of the first surface and the said folding section is provided with the unfolded extended area that can be unfolded at least in the lateral direction by folding the one side edge of the sheet 32 towards inside so that inner sides face to each other in such a manner the third folding line c is formed at the takeoff opening 38 of the first surface as a folding back point. Further, the dimension of the unfolded extended area is increased by extending the one side edge forming the folding section existing inside of the first surface to the second surface exceeding the first folding line a or the third surface exceeding the second folding line b. By this, since the hand holding the wrapping material is covered more securely, even if an used interlabial pad is put on the wrapping material in haste, menstrual blood absorbed does not adhere to the hand resulting in easier and securer discard. In addition, if the folding section existing inside of the first surface shown above is bonded in such a manner the forward and rear end can not be peeled off to make the pocket portion 34, the interlabial pad can be inserted smoothly even if the used interlabial pad is slid obliquely to the inlet of the pocket portion 34 since the depth of the pocket portion is larger.

However, if the one side forming the folding section exceeds the unsealing section 33 at the one edge opposite to the second folding line of the third surface, a wearer may be confused because there are two unsealing sections. So, this situation is unfavorable. In order to evade this situation, as shown in Fig. 23 (b), if the first surface (1) is overlapped on the upper surface of the third surface (3), the third folding line c becomes the unsealing section 53 and the other side edge forming the folding section exceeding the one side edge that was originally the unsealing section becomes the takeoff opening 58 of the interlabial pad 10. By this, the confusion of a wearer shown above can be evaded.

As described above, the maximum unfolding dimension when the unfolded extended section and the pocket portion in the folding area are unfolded in the lateral direction of the wrapping material may be at least 200% or more on the basis of the apparent lateral direction of the wrapping body, preferably 210 to 500%, more preferably 250 to 350%.

In the area where the first surface and the third surface are overlapped, invasion of dust and dirt into the individual wrapping body from the unsealing section when taking it along is prevented by applying an adhesive in the longitudinal direction continuously or intermittently resulting in hygienic storage of the interlabial product. In this case, if the position where an adhesive is applied is provided at the position shifted by 1 to 10mm to the second folding line b from the one side edge, unsealing becomes easier since the unsealing section has a dry edge. An adhesive to be applied is selected from the re-sealable hot melt type adhesives without particular limitation. It is applied by any one of application patterns such as sheet pattern, banded pattern, spiral pattern, dot pattern known in the art.

According to the present invention, the wrapping body 61 may be provided with the fixing tape 67 at the unsealing section 63 as shown in Fig. 24. In this case, it is possible to make the sealing state securer after the used interlabial pad is enclosed by unfolding the wrapping container.

### [Interlabial pad]

### <Shape>

The shape of the interlabial pad contained in the wrapping body is not particularly limited so long as it fits the labia of females readily. Considering that the shape of labia of females is substantially symmetrical and longitudinal, it is preferable that the shape of the pad is substantially longitudinal. Oval shape, gourd shape, drop shape, etc. are desirable.

It may have a three-dimensional structure where a projection such as convex in order to contact the labia securely.

Further, it may be provided with a mini sheet to form a finger inserting hole on the garment-facing side. With this type of interlabial pad, the position of the ostium vaginae as a concave portion is detected with a sensitive fingertip by inserting a finger in such a way that the fingerprint side of the finger contacts the backside of the sheet. Thus, it is possible to fix an interlabial pad so that the ostium vaginae is plugged up securely preventing menstrual blood from leaking. In addition to that, the finger does not contact the upper and lower surface of the interlabial pad that contacts the inner wall of the labia resulting in hygienic fixing into the interlabial space. In order to contain this type of interlabial pad, as shown in Fig. 25, the interlabial pad 10 is preferably contained in such a way that the mini sheet piece 14 is positioned near the unsealing section 73. By this, a wearer can find the mini sheet piece 14 attached to the interlabial pad 10 as soon as she unsealed the unsealing section 73. Thus, she can immediately remove the interlabial pad 10 from the wrapping container by inserting a finger into the finger inserting opening resulting in simple preparation for fixing.

### <Size>

The longitudinal dimension of the interlabial pad may be 50 to 200mm, preferably 80 to 150mm. The apparent lateral dimension thereof seen from the body-facing side may be 10 to 80mm, preferably 20 to 40mm.

If a projection such as convex shape is formed on the body-facing side, the height of the projection may be 5 to 30mm, preferably 10 to 20mm. If the dimension is smaller than the range, the maximum absorption capacity of the interlabial pad is readily exceeded resulting in outflow of menstrual blood. On the other hand, if the dimension is larger than the range, it not only exceeds the size of sanitary napkins, etc. used at the same time but also may deteriorate wearing feeling. In addition, if the height of the projection is smaller than the range, the pad does not fit the labia tightly. On the other hand, the height of the projection is larger than the range, it exceeds the depth of labia resulting in foreign-body sensation of a wearer.

The state of the interlabial pad contained in the wrapping body is not particularly limited. However, considering compactness when taken along and prevention of menstrual blood from outflowing by tightly fitting the interlabial pad that is longitudinal to the labia symmetrically, the interlabial pad is preferably folded at least substantially along the center line in the longitudinal direction.

The apparent dimension in the longitudinal direction of the interlabial pad contained in the wrapping body is preferably 80 to 150mm. The substantial apparent dimension thereof in the lateral direction is preferably 10 to 30mm.

The apparent length dimension of the wrapping body is preferably 105 to 130% of the dimension of the interlabial pad in the longitudinal direction. The apparent dimension in the lateral direction is preferably 105 to 130% of the dimension in the lateral direction of the interlabial pad folded along the center line in the longitudinal direction. If the dimensions are smaller than the range shown above, it is hard to smoothly remove the interlabial pad from the wrapping container and a wearer feels it bothersome. If the dimensions exceed the range, the compactness when taken along can not be secured.

### <Application>

The interlabial pad contained in the wrapping body can be used for absorbing excretion (vaginal discharge) secreted from the ostium vaginae other than menstrual blood. And it can be used for incontinence protection, i.e., absorbing urine discharged from the urethral opening situated between labia same as the ostium vaginae.

### [Construction materials of the wrapping container]

### <Sheet>

Materials known in the art can be used for the sheet used in the wrapping container. Examples usable herein include: polyethylene, polyrulopyrene polyester, polyvinyl alcohol, polylactic acid, polybutylene succinate, nonwoven fabrics, paper and laminated materials thereof with thickness of 15 to 60 micron. Specifically, films composed mainly of LDPE (low density polyethylene) adjusted in the range of a specific weight per unit area of 15 to 30g/m² are generally used.

Preferred examples include laminate materials where nonwoven fabrics or paper whose interior surface is composed of a film considering liquid impermeability. Specifically, a compound material where a film mainly composed of LDPE adjusted in the range of a specific weight per unit area of 5 to 20g/m² is laminated on one side of a compound nonwoven fabric constituted by spun bond-meltblown-spun bond of 6 to 10gsm-5 to 20gsm-6 to 10gsm can be used.

If flushability is considered, nonwoven fabrics or films composed of biodegradable polylactic acid, polybutylene succinates, polylactic acid, polyisocyanates, starch, etc. can be used. If water-solubility is considered, films composed mainly of polyvinyl alcohol as well as lavatory paper can be used. Specifically, a laminate body where a film of polyvinyl alcohol adjusted in the range of a specific weight per unit area of 5 to 10g/m² and a water-soluble paper adjusted in the range of a specific weight per unit of 15 to 30g/m² can be used.

The sheet can be used for discarding the used interlabial pad after use by wrapping the same. In this case, it is desirable that menstrual blood adhered to or absorbed by the interlabial pad does not flow out of the sheet. In other words, it is desirable that menstrual blood does not adhere to fingers when gripping the sheet containing the used interlabial pad. So, materials with favorable liquid impermeability, for example, films composed of polyethylene, polypropylene, etc. with thickness of about 15 to 60µm, or laminate materials where the said film materials are laminated on nonwoven fabrics or paper are preferably used.

It is also desirable that the sheet shields the color of menstrual blood adhered to or absorbed by the interlabial pad. In order to obtain such a shielding effect, for example, an ink with a pigment of 5 to 40% by weight, a resin of 10 to 20% by weight and a solvent of 40 to 85 % by weight may be photogravured, etc. on the wrapping sheet, the sheet material may be opacified in advance by mixing a pigment from 0.2 to 10 % by weight on the basis of a raw resin material in the wrapping sheet or the wrapping sheet may be dyed. Pigments and dyes to be used may be white or colored. For example, basic dyes usable herein include C.I Basic Violet 3, etc., vat dyes include C.I. Vat Blue 1, etc., acid dyes include Blue 1, Blue 2, C.I. Acid Red 51, etc., direct dyes include C.I. Direct Yellow 12, C.I. Direct Orange 26, C.I. Direct Violet 51, C.I. Direct Blue 1, C.I. Direct Red 23, etc., reactive dyes include C.I. Reactive Orange 16, C.I. Reactive Black 5, C.I. Reactive Blue 21, C.I. Reactive Red 21, etc., inorganic pigments include titanium oxide, titanium yellow, calcium carbonate, Carbon Black, cobalt blue, etc., organic pigments include Yellow 401, Orange 204, Blue 404, Red 201, C.I. Pigment Yellow 14, C.I. Pigment Green 7, C.I. Pigment Violet 19, C.I. Pigment Blue 27, C.I. Pigment Red 166, etc. They may be used alone and independently or be combined with two or more of them as required.

### <Fixing tape>

Fixing tapes usable herein include, but not limited to, a film layer where an adhesive is applied to the single side, or, if flushability is considered, a tissue paper where an adhesive layer is applied to the single side of the tissue paper, etc. that are known in the art.

### [Viable cell count suppressing treatment]

From a hygiene point of view, the wrapping container according to the present invention, especially, the parts to contact the interlabial pad is preferably treated with a treatment to suppress the viable cell count. Viable cell count suppressing treatments include: sterilization process or germicidal treatment during manufacturing, use of antibacterial materials for individual wrapping containers, etc.

### [Construction materials of the interlabial pad]

### <Water permeable sheet>

Materials that are liquid hydrophilic and do not apply stimulus to skin are usable for water permeable sheets provided at the body-facing side of the interlabial pad. Materials usable herein include nonwoven fabrics used alone or in combination manufactured by methods such as melt blown, spun bond, point bond, through air, needle punch, wet spun lace, foam film.

A fibrous sheet may be made from either a single fiber or a complex fiber made into a sheet, the fibers being made from either alone of rayon, acetate, cotton, pulp or synthetic resins or in combination thereof compounded to form a core-sheath structure.

Among the materials, considering the liquid mobility from the inner face of the labia, chemical stimulation by an activator, and adhesion with the inner wall of the labia, it is preferable to laminate rayon with 1.1 to 4.4 dtex fineness and 7 to 51 mm fiber length by 40 to 80 % of a total specific weight per unit area on the body face side, and to laminate a mixture of rayon with 1.1 to 4.4 dtex fineness and 7 to 51 mm fiber length by 14 to 42 % of a total specific weight per unit area and PET with 1.1 to 4.4 dtex fineness and 7 to 51 mm fiber length by 6 to 18 % of a total specific weight per unit area on the garment face side. After laminating them so that the total specific weight per unit area of the two layers becomes 20 to 60 g/m², the fibers are entangled by water- flow interlacing treatment and then dried to prepare spun lace nonwoven fabric with the thickness of 13 to 0.50 mm. The spun lace nonwoven prepared as described is preferable. At this time, by mixing PET on the garment face side, bulkiness can be easily maintained even if the permeable sheet becomes wet. Therefore, adhesion between the inner wall of the labia can be maintained.

### <Absorbent body>

Materials to be used for the absorbent body contained in the interlabial pad include pulp, chemical pulp, rayon, acetate, natural cotton, water-absorbent polymer, fibrous water-absorbent polymer, synthetic fiber. They may be used alone or as a mixture of two or more.

A mixture blended as required is made into a sheet by technologies such as pressure bond by embossing process, interlacing by needling that are well known in the art. The sheet may be adjusted by bulking, layering, folding, etc. as required.

Materials for the sheet may be used in a sheet shape or may be used by processing the same into powder. Their usages are not limited.

It is preferable for the absorbent body, although any material can be used as long as it is capable of absorbing and holding liquid (body fluid), to be bulky, hard- to- be deformed, less chemically stimulant, and highly flexible to fit into the labia. Specifically, a nonwoven sheet in which, 50 to 150 g/m² of pulp selected from the range of the fiber length of 1 to 10 mm is laminated on the garment face side and, on the body face side, 150 to 250 g/m² of a mixture obtained by mixing 60 to 90 % of rayon with 1.1 to 4.4 dtex fineness and 20 to 51 mm fiber length with 40 to 10 % of natural cotton by this mixing ratio is laminated, which then to be formed into a sheet by dotted embossing to have 2 to 10 mm bulkiness, and more preferable to have 3 to 5 mm bulkiness. Thereby, liquid can be easily transmitted from the body face side to the garment face side resulting in the improvement of the absorbing and holding capacity. Furthermore, by providing a mesh spun lace nonwoven fabric of rayon with 1.1 to 4.4 dtex fineness and 25 to 51 mm fiber length by a specific weight per unit area of 15 to 40 g/m², the liquid transmitted from the body face side can be dispersed by the mesh spun lace to be induced to almost all over the region of the pulp layer. Therefore, more liquid can be effectively absorbed.

### <Water impermeable sheet>

Materials that can prevent menstrual blood retained in the absorbent body from getting out of the interlabial pad can be used for water impermeable sheets for interlabial pad. If they are moisture permeable materials, it is possible to reduce humidity and unpleasantness during wearing the interlabial pad.

Such materials include, for example, a sheet film wherein a synthetic resin is transformed into a membrane, an air permeable film made by filling an inorganic filler and performing a pulling process, a laminate material wherein a paper or an unwoven fabric and a film are combined, an air permeable/liquid impermeable sheet with pore area rate of 10 to 30% and porous diameter of 0.1 to 0.6mm made by arranging capillaries so that they are headed to the absorbent body, etc.

Further, when considering flexibility not spoiling wearing feeling, for example, a film having a specific weight per unit area of 15 to 30g/m² composed mainly of low density polyethylene (LDPE) having density of 0.900 to 0.925g/cm³ is preferably used. More preferably, the said film is emboss processed to reduce contact ratio and friction resistance by providing convex bosses in order to reduce a risk that the interlabial pad falls off from the labia due to a large friction caused by contact with other impermeable sheets, pads used at the same time, underwear, etc. when the pad is worn between the labia.

### <Adhesives>

It is possible to apply an adhesive on the body-facing side of the interlabial pad in order to promote fixing to the interlabial space. Adhesives for bonding to skin include water-soluble polymers, crosslinking agents, plasticizers and gel adhesives composed of water, etc. More specifically, examples of water-soluble polymers include gelatin, sodium polyacrylic acid, polyvinyl alcohol, carboxymethyl cellulose, etc. Examples of crosslinking agents generally include water-soluble metal salts such as calcium chloride, magnesium sulfate. Examples of plasticizers include: glycerin, wax, paraffin, etc.

Besides them, it is possible to use pressure sensitive hot melts to form adhesive areas. Pressure sensitive hot melts are obtained by melting and blending adhesiveness granting agents such as terpene resins, rosin resins and plasticizers such as waxes with synthetic rubber resins with synthetic rubbers such as styrene-butadiene-styrene block copolymers (SBS), styrene-isoprene-styrene block copolymers (SIS), styrene-ethylene-butadiene-styrene block copolymers (SEBS), styrene-ethylene-propylene-styrene block copolymers (SEPS) as a main component.

Further, it is also possible to use silicon resin adhesives. Example of silicon resin adhesives include mixtures composed mainly of silicon resins and fluorocarbon resins, mixed with crosslinking agents such as platinum, molybdenum, antimony, plasticizers such as esters waxes, glycerin, machine oils.

As shown above, there are varied types of adhesives to form adhesive areas. However, if stability of application is considered, pressure sensitive hot melts are preferred. Examples of heat sensitive hot melt adhesives with higher application stability include the one where SEBS of 15 to 25% by weight, a plasticizer of 15 to 35% by weight and an adhesiveness granting agent of 40 to 70% by weight are melted and blended. Antioxidants, fluorescence inhibitors of 0.1 to 1.0% by weight may be added to this kind of heat sensitive hot melt adhesives.

### <Mini sheet piece>

Materials that are the same as the said water permeable sheets or the water impermeable sheets may be used for the mini sheet piece. Materials with extensity or elasticity at least in the lateral direction are preferably used.

By using such materials for the mini sheet piece, even if the size of a fingertip of a wearer is larger than the specified finger inserting opening, the interlabial pad according to the present invention can be used effectively irrespective of the fingertip size of a wearer because the mini sheet piece at least stretch in width direction.

Basically, materials with elasticity include: for example, synthetic rubbers such as styrene-butadiene-styrene block copolymers (SBS), styrene-isoprene-styrene block copolymers (SIS), urethane; films made from amorphous olefin resins with density selected from 0.88 to 0.900g/cm³, porous foam films, nets. Woven fabrics and woven fabrics wherein a fiber spinning filament made from a synthetic rubber is woven into can be also used. Further, spun bond nonwoven fabrics and meltblown nonwoven fabrics composed mainly of a synthetic rubber and a foamed sheet can be also used.

Considering flexible feeling when wearing, a porous foam film made from SEBS adjusted to thickness from 15 to 40 µ, pore area from 0.28 to 1.77mm², pore area rate from 40 to 70% is preferably used.

Nonwoven fabrics include: spun lace nonwoven fabrics made from complex synthetic fibers such as PE/PP, PE/PET, PP/PP having thermal shrinkage property, wherein core component has a high melting point and the sheath component has a lower melting point, and fibers are interlaced by water flow pressure; shrink type nonwoven fabrics wherein shrinkage of fibers are accelerated by performing hot air reprocessing; so called extensible spun bonds wherein continuous long fibers are applied with tentering after they are made into a sheet by heat seal.

More specifically, preferable materials rich in flexibility and drape feeling include shrink type nonwoven fabrics made from compound synthetic fiber such as PE/PP, PE/PET, PP/PP with fineness in the range from 1.1 to 4.4 dtex and the length in the range from 7 to 51 mm, having thermal shrinkage property, wherein the core component has a high melting point and sheath component has a lower melting point, adjusted in the range of a specific weight per unit area of 10 to 60g/m². Further, laminated types of the said materials can be also used.

Non-extensible materials that is applied with extensity before use included: among nonwoven fabrics, through air nonwoven fabrics made from complex synthetic fibers such as PE/PP, PE/PET, PP/PP having thermal shrinkage property, wherein core component has a high melting point and the sheath component has a lower melting point; spun lace nonwoven fabrics wherein fibers are interlaced by water pressure; spun bond nonwoven fabrics transformed into a sheet by layering continuous fibers; needlepunch nonwoven fabrics wherein fibers are interlaced with one another by needles; SMS nonwoven fabrics wherein spun bond and meltblown are layered in multiple layers to form a sheet; porous foam films; films mainly composed of PE resins. They may be used either alone or in combination of two or more.

Further, the said materials can be applied with extensity by corrugate processing wherein materials are fit between the male and female dies and a shape is embossed by heat, temperature and pressure. More specifically, throughair nonwoven fabrics composed mainly of complex synthetic fibers with fineness adjusted to the range from 1.1 to 4.4 dtex and adjusted to the range of a specific weight per unit area of 10 to 60g/m², corrugated so that they can be extended in the lateral direction are preferred. It is desirable that corrugate processing is performed in such a way that male and female dies are provided so that extensity is at least 10%, more preferably, 20 to 50%, still further preferably, having a behavior in the range of 0.01 to 0.05N/25mm of loads when 30% extended (test condition: Tensilon tensile tester, speed, 100mm/min, chuck interval, 100mm). Other methods to apply extensity include cut lines, circular cutouts.

### [Configuration of the interlabial pad applied with biodegradability, water dispersibility and water solubility]

### <Wrapping container>

The construction materials of the wrapping material constituting the wrapping body according to the present invention may be biodegradable materials and/or water soluble materials and/or water dispersible materials. If the materials of the wrapping container are water-soluble materials or water-dispersible materials, and the materials of the used interlabial pad to be wrapped in the container are also water-soluble materials or water-dispersible materials, a wearer can discard the pad wrapped in the container into lavatory bowls as it is after use. Thus, she is relieved of the burden to discard wrapping containers and amounts of wastes in bath rooms can be reduced.

Specific examples of these wrapping containers include: a compound material where a tissue adjusted of a specific weight per unit area of 15 to 40g/m² and a polyvinyl alcohol with a specific weight per unit area of 20 to 50g/m² are laminated, silicon is applied to the side of polyvinyl alcohol in the range of 0.5 to 1µm, a spun bond nonwoven fabric composed mainly of a polylactic acid fiber adjusted of a specific weight per unit area of 15 to 40 g/m².

In this Specification, "biodegradability" means that a substance is decomposed into gas such as carbon dioxide or methane, water, and biomass under anaerobic or aerobic condition according to the natural process under the existence of bacteria represented by actinomycetes and other microbes, and also means that the biodegradability (biodegradable rate and biodegradable degree) of the substance equals to a material naturally generated such as fallen leaves or a synthetic polymer generally recognized having the same biodegradability under the same environment. "Water dispersibility" has the same meaning as water degradability. It means a characteristic in which, while having no influence when used in a limited amount of moisture (menstrual blood), in a large amount of water or water current, the fabric is easily dispersed into small pieces at least to a degree where an ordinal toilet plumbing is not clogged. "Water dispersibility" means the same as water degradability, where there is no effect from the limited amount of water (menstrual blood) upon use, whereas in conditions of large amounts of water or under water flow, the fibers are easily dispersible into at least small pieces which cannot clog the toilet plumbing. "Water solubility" means the property of not being affected by limited amount of water (menstrual blood) upon use, but being soluble in large amounts of water or under a flow of water.

### <Construction of the interlabial pad>

The interlabial pad contained in the wrapping container shown above is also preferably composed of biodegradable materials and/or water dispersible materials and/or water soluble materials. These kinds of interlabial pads can be fallen off into lavatory bowls as they are after use. So, pads can be discarded easily and cleanly. In addition, wastes in bath rooms can be further reduced:

### <Water permeable sheet >

Materials that can be used for water permeable sheets include: wet forming spun lace nonwoven fabrics with fiber length selected from 1 to 15mm. Other materials include hydrolyzed biodegradable resins such as polylactic acid, polybutylene succinate. Examples usable herein include meltblown nonwoven fabrics made from polylactic acid adjusted to the range of a specific weight per unit area of 20 to 60g/m² and spun bond nonwoven fabrics adjusted to the range of a specific weight per unit area of 15 to 30g/m² and fineness adjusted to the range of 1.1 to 3.3 dtex. Materials for nonwoven fabrics may be either applied with pore opening treatment or not.

For other materials, it is possible to use acetate or a synthetic fiber alone, or a tow that is a continuous fiber composed of laminated bodies, by adjusting it in the range of a specific weight per unit area of 50 to 300g/m² and fibrillating fibers thereof.

### <Absorbent body>

Nonwoven fabric sheets obtained by needling can be used for absorbent bodies. It is desirable to use carboxymethyl cellulose fibers considering biodegradability of polymer absorbent materials.

### <Water impermeable sheet >

Materials usable for water impermeable sheets include laminated papers where tissues are laminated with PVA films, film sheets where one or both sides of, or partial surface of PVA film is water-repellent by applying silicon, etc., PVA films mixed with silicon, starch films, films made from biodegradable resins of hydrolyzed polylactic acid, polybutylene succinates, etc. If required, inorganic pigments may be mixed by 0.1 to 5% to color.

It is desirable to use laminated papers where films made from polylactic acid are laminated with thickness of 10 to 20µ with tissues selected from the range of a specific weight per unit area of 15 to 20g/m² and further composite area ratio when laminated being 5 to 40% considering maintenance of leakage resistance under hyper humidity and lower loads to septic tanks.

### <Mini sheet piece>

Materials usable for the mini sheet piece include: films, spun bond nonwoven fabrics, meltblown nonwoven fabrics, etc. made from biodegradable materials such as polylactic acid, polybutylene succinate; films and nonwoven fabrics, etc. made from water soluble materials such as PVA, CMC; water dispersible tissues, spun lace nonwoven fabrics, etc. composed mainly of cellulose fibers, recycled cellulose fibers, etc.

Of these, spun bond non woven clothes or meltblown nonwoven fabrics composed mainly of biodegradable materials that are made into sheets whose fineness is adjusted to the range from 0.1 to 3.3 dtex, adjusted to the range of a specific weight per unit area of 15 to 40 g/m², obtained from the said mechanical corrugate processing are preferred.

### < Bonding methods >

Bonding methods include: bonding with polyvinyl alcohol, etc. with water solubility or water swellingness, heat seal bonding, hydrogen bonding, etc. They are used alone or two or more of them are used at the same time.

### Industrial Applicability

As described above, the individual wrapping container for the interlabial pad according to the present invention can be unfolded to make a discarding member with large size. So, the used interlabial pad can be covered with this discarding member resulting in clean and smooth discard of the used interlabial pad.

## Claims

1. A wrapping body comprising:
an interlabial pad worn between labia; and
an individual wrapping container for wrapping individually each one of said interlabial pad,
wherein said individual wrapping container is provided with an unwrapping portion for unwrapping,
wherein said container has a shape developable from the unwrapping portion, and
wherein said container has an enough size to enclose a used interlabial pad in a developed state.

2. The wrapping body according to claim 1,
wherein said individual wrapping container comprises a series of wrapping sheet superposing each other, the sheet comprising two ends and being wrapped in a way to form an overlapping part where the both ends of the wrapping sheet and a vicinity of the both ends are overlapped each other;
wherein said unwrapping portion is formed to unwrap said individual wrapping container by releasing an overlapping state of the overlapping part; and
wherein said wrapping sheet turns to be a waste discarding sheet for enclosing a used interlabial pad in a state that said wrapping container is developed after unwrapping said individual wrapping container.

3. The wrapping body according to claim 2, wherein said wrapping sheet has fine protrusions on a surface of at least one face of said wrapping sheet.

4. The wrapping body according to claim 2 or 3, wherein said wrapping sheet comprises an inner face to be in contact with the interlabial pad to be wrapped and an outer face to be held by the wearer, the wrapping body comprising
a bent portion bent toward a side of said inner face provided in a vicinity of one end of the two ends of said wrapping sheet in the longitudinal direction,
wherein said bent portion forms a pocket portion capable of receiving said used interlabial pad.

5. The wrapping body according to claim 4, wherein said wrapping sheet is folded in a way to have one or more folded portions where a part of said wrapping sheet is bent toward a side of said inner face near an end opposite to the end where said pocket portion is provided;
wherein said used interlabial pad can be wrapped with said waste discarding sheet including, as a part of said waste discarding sheet, said folded portions that are developed in a state after said individual wrapping container is unwrapped and developed; and
wherein said pocket portion can receive said used interlabial pad that is wrapped with said part of said waste discarding sheet.

6. The wrapping body according to claim 4 or 5, wherein a dimension of an opening of said pocket is longer than a dimension of a bottom of said pocket.

7. A wrapping body comprising:
an interlabial pad worn between labia; and
an individual wrapping container for wrapping individually each one of said interlabial pad,
wherein said individual wrapping container is provided with an unwrapping portion for unwrapping, and a shape developable from the unwrapping portion; and
wherein said individual wrapping container comprises an elongated area; and
wherein said individual wrapping container turns to be of a size to enclose a used interlabial pad in a developed state by extending said elongated area.

8. The wrapping body according to claim 7, wherein said elongated area is formed by an emboss processing.

9. The wrapping body according to any one of claims 1 to 8, wherein said unwrapping portion of said individual wrapping container is provided with a sealing means that is re-sealable.

10. The wrapping body according to any one of claims 1 to 9,
wherein said interlabial pad is contained in a bent state before said individual wrapping container is developed; and
wherein said used interlabial pad is contained in a developed state when said individual wrapping container is developed to enclose said used interlabial pad.

11. The wrapping body according to any one of claims 1 to 10,
wherein said interlabial pad is provided with a finger insertion opening, in which a finger can be inserted, on an opposite side face to a body side face; and
wherein said finger insertion opening is disposed near said unwrapping portion of said individual wrapping container; and
wherein the finger insertion opening of said contained interlabial pad can be exposed naturally at a beginning of unwrapping the individual wrapping container when said individual wrapping container is unwrapped.
